(19) Europäisches Patentamt
European Patent Office
Office européen des brevets

(11) Numéro de publication: **0 110 754**
**A1**

(12) **DEMANDE DE BREVET EUROPEEN**

(21) Numéro de dépôt: **83402133.9**

(22) Date de dépôt: **02.11.83**

(51) Int. Cl.³: **C 07 C 103/183**, C 07 C 102/08

(30) Priorité: **02.11.82 FR 8218313**

(43) Date de publication de la demande: **13.06.84**
**Bulletin 84/24**

(84) Etats contractants désignés: **BE CH DE FR GB IT LI LU NL**

(71) Demandeur: **DESHORS, 38 bis, Rue d'Artois, F-75008 Paris (FR)**

(72) Inventeur: **Lhommet, Gérard, Place de Coeuilly, F-94500 Champigny-sur-Marne (FR)**
Inventeur: **Allaigre, Jean-Pierre, 12, rue Danton, F-95460 Ezanville (FR)**
Inventeur: **Celerier, Jean-Pierre, 80, Boulevard de Reuilly, F-75012 Paris (FR)**
Inventeur: **Graffe, Bernadette, 22, rue d'Orléans, F-92200 Neuilly-sur-Seine (FR)**
Inventeur: **Sacquet, Marie-Claude, 84, Avenue du Général de Gaulle, F-94700 Maisons-Alfort (FR)**

(74) Mandataire: **Koch, Gustave et al, Cabinet PLASSERAUD 84, rue d'Amsterdam, F-75009 Paris (FR)**

(54) **Nouveau procédé de synthèse du chlorhydrate de 4-aminobutyramide.**

(57) Selon l'invention, on prépare le chlorhydrate de 4-aminobutyramide par hydrolyse ménagée du chlorhydrate de 4-aminobutyronitrile.

Cette hydrolyse peut être effectuée dans un acide aqueux ou anhydre, en présence d'acide chlorhydrique aqueux ou de gaz chlorhydrique anhydre.

La réaction est avantageusement effectuée à une température de 15 à 55°C.

Le produit est isolé par désolubilisation, éventuellement précédée d'une concentration du milieu réactionnel.

EP 0 110 754 A1

NOUVEAU PROCEDE DE SYNTHESE DU CHLORHYDRATE DE 4-AMINO-BUTYRAMIDE.

L'invention a pour objet un nouveau procédé de fabrication du chlorhydrate de 4-aminobutyramide ou gabamide.

Le gabamide, de par ses propriétés psychotropes, présente un intérêt à lui seul, et en tant que matière première dans la synthèse de molécules à structure benzylidénique ayant des propriétés gabamimétiques.

On connaît déjà plusieurs procédés de préparation du gabamide, à savoir notamment :

- à partir du N-(2-hydroxyéthyl)phtalimide, par l'intermédiaire de l'acide 4-aminobutyrique [Acta Polon. Pharm 23(2), 111 (1966)] ;

- par hydrogénation catalytique du 3-cyano-propionamide, lui-même obtenu à partir de l'acrylamide et de l'acide cyanhydrique ou d'un cyanure alcalin (Angew. Chem. 92(8) 640 (1980) et EP-A-29890) ;

- par réduction catalytique du 4-nitrobutyramide en milieu acide (FR-A-2 497 797) ; et

- à partir de la benzylamine et du 4-chlorobutyronitrile, par l'intermédiaire du N-benzylamino-4-butyronitrile et du N-benzylamino-4-butyramide [Arch. Pharm. 314(7), 648 (1981) et EP-A-56 082].

Ces méthodes sont en général longues et coûteuses, voire même dans certains cas dangereuses (utilisation d'acide cyanhydrique ou d'un cyanure alcalin).

Au contraire, l'invention fournit un procédé simple et économique de préparation du chlorhydrate de gabamide avec une bonne pureté et de bons rendements.

En effet, de manière tout à fait surprenante, selon l'invention on a trouvé que le chlorhydrate de 4-aminobutyramide peut être obtenu à partir du chlorhydrate de 4-amino-butyronitrile, par simple hydrolyse.

Plus précisément, le procédé de préparation du chlorhydrate de 4-aminobutyramide, ou gabamide, selon l'invention est caractérisé en ce qu'il consiste essentiellement à effectuer une hydrolyse ménagée du chlorhydrate de 4-amino-butyronitrile de formule :

$$H_2N-CH_2-CH_2-CH_2-CN,\ HCl.$$

On entend ici par "hydrolyse ménagée" une hydrolyse réalisée dans des conditions suffisamment douces, notamment de température, pour qu'elle ne conduise pas à l'acide correspondant.

Il est très surprenant, compte tenu des connaissances de l'homme du métier en la matière, et en particulier de l'art antérieur cité plus haut, qu'une telle hydrolyse conduisant du nitrile à l'amide puisse être effectuée sans réaction secondaire gênante telle que cyclisation, et ceci bien que la fonction amine ne soit pas protégée.

Le chlorhydrate de 4-aminobutyronitrile est décrit dans la littérature et peut être obtenu par différentes voies chimiques connues.

Il peut être notamment préparé par ammolyse d'un 4-halogénobutyronitrile, par exemple le 4-chlorobutyronitrile, selon le schéma suivant :

$$Cl-CH_2-CH_2-CH_2-CN \xrightarrow[t^{re}\ \text{ambiante}]{NH_3\ \text{liquide}} H_2N-CH_2-CH_2-CH_2-CN + NH_4Cl$$

$$\downarrow HCl$$

$$H_2N-CH_2-CH_2-CH_2-CN,\ HCl$$

Cette méthode est décrite dans le brevet US 2 443 292 et dans J. Org. Chem. 33(5) 2109 (1968).

Le 4-aminobutyronitrile, que l'homme du métier peut aisément, sur la base de ses connaissances usuelles en la matière, transformer en chlorhydrate, peut aussi être préparé

- par l'intermédiaire du 4-phtalimidobutyronitrile, préparé à partir d'un halogénobutyronitrile [J. Am. Chem. Soc. 81, 4328 (1959) et J. Chem. Soc. 1369 (1947)] ;

- par hydrogénation catalytique ménagée du succinodinitrile (brevets IT 845 999 et 848 757).

L'hydrolyse ménagée du chlorhydrate de 4-aminobutyronitrile peut être effectuée dans un acide, aqueux ou anhydre, en présence d'acide chlorhydrique ou de gaz chlorhydrique anhydre.

Selon un mode de réalisation de l'invention, l'hydrolyse est effectuée dans de l'acide chlorhydrique aqueux concentré, en l'absence de tout autre acide.

Selon un autre mode de réalisation de l'invention, l'hydrolyse est effectuée dans un acide tel que notamment l'acide formique, l'acide acétique ou l'acide propionique, seul ou en mélange, anhydre ou non, en présence d'acide chlorhydrique aqueux ou de gaz chlorhydrique anhydre.

La température de la réaction se situe de préférence dans la gamme de 15 à 55°C.

La durée de la réaction dépend dans une large mesure des conditions opératoires et en particulier de la température choisie. A titre indicatif, il est mentionné que cette durée peut aller de 60 heures à 15°C à environ 10 heures à 55°C.

Le chlorhydrate de 4-aminobutyramide ainsi préparé peut être isolé selon les techniques courantes connues de l'homme du métier pour isoler les produits de ce type, telles que l'addition d'un agent désolubilisant comme l'isopropanol ou l'acétone, ou un mélange des deux.

Il est dans certains cas avantageux d'effectuer, avant désolubilisation, une concentration partielle du milieu sous pression réduite, afin de diminuer la quantité de solvant nécessaire à cette désolubilisation.

L'invention sera mieux comprise à l'aide des exemples illustratifs et en aucune manière limitatifs qui suivent.

Exemple 1

12,05 g de chlorhydrate de 4-aminobutyronitrile sont introduits dans un erlenmeyer de 500 ml contenant 30 ml d'acide chlorhydrique à 22° Bé (d = 1,19). Le mélange est

maintenu pendant 40 heures à une température comprise entre 25 et 30°C.

La solution est ensuite refroidie entre 0 et +5°C puis additionnée de 120 ml d'isopropanol et de 120 ml d'acétone.

Après trois heures à cette température, les cristaux de chlorhydrate de 4-aminobutyramide sont filtrés puis lavés par 20 ml d'acétone. Après séchage, on recueille 10,8 g de cristaux, soit un rendement de 78 %.
Point de fusion : 137-138°C.
Les spectres IR et RMN sont conformes à la structure du produit recherché.

Exemple 2

Une solution de 6,02 g de chlorhydrate de 4-aminobutyronitrile dans 15 ml d'acide chlorhydrique à 22° Bé (d = 1,19) est portée entre 40 et 45°C puis maintenue 18 heures à cette température.

La solution obtenue, traitée dans les conditions de l'exemple 1, conduit à 5,67 g de cristaux de chlorhydrate de 4-aminobutyramide.
Point de fusion : 138°C.
Les spectres IR et RMN sont conformes à la structure du produit recherché.

Exemple 3

12,05 g de chlorhydrate de 4-aminobutyronitrile sont mis en suspension dans 50 ml d'acide formique à 80 %. On ajoute 25 ml d'acide chlorhydrique à 22° Bé (d = 1,19) puis on porte le mélange à 40°C, pendant 15 heures.

La solution est refroidie puis concentrée sous pression réduite à la moitié de son volume initial.

On ajoute 100 ml d'acétone. La suspension obtenue est filtrée et les cristaux sont séchés en étuve.

On obtient 10,4 g de chlorhydrate de 4-aminobutyramide, soit un rendement de 75 %.
Point de fusion : 135-137°C.
Les spectres IR et RMN sont conformes à la structure du produit recherché.

Exemple 4

6,02 g de chlorhydrate de 4-aminobutyronitrile sont introduits dans un erlenmeyer de 250 ml contenant 25 ml d'acide acétique glacial et 12,5 ml d'acide chlorhydrique à 22° Bé (d = 1,19). La solution obtenue est maintenue 10 heures à 55°C.

Le milieu réactionnel, traité dans les conditions de l'exemple 3, fournit 5 g de cristaux de chlorhydrate de 4-aminobutyramide, soit un rendement de 72 %.
Point de fusion : 137-138°C.
Les spectres IR et RMN sont conformes à la structure du produit recherché.

Exemple 5

18,1 g de chlorhydrate de 4-aminobutyronitrile sont mis en suspension dans 200 ml d'acide acétique glacial. On porte le mélange à 45°C puis on y fait barbotter un léger courant de gaz chlorhydrique anhydre.

Après 8 heures de réaction, le courant de gaz chlorhydrique est interrompu. Le milieu est concentré au tiers de son volume. Après addition de 150 ml d'acétone, le mélange est filtré et les cristaux séchés. On obtient ainsi 16,2 g de chlorhydrate de 4-aminobutyramide, soit un rendement de 78 %.
Point de fusion : 135-137°C.
Les spectres IR et RMN sont conformes à la structure du produit recherché.

Exemple 6

12,05 g de chlorhydrate de 4-aminobutyronitrile sont introduits dans un erlenmeyer de 500 ml contenant 150 ml d'acide propionique et 50 ml d'acide chlorhydrique à 22° Bé (d = 1,19).

Le mélange est agité pendant 40 heures à 25°C. La solution obtenue est concentrée sous pression réduite au quart de son volume initial. Le concentrat est repris par 150 ml d'acétone. Le mélange est filtré et les cristaux sont séchés en étuve. On recueille 11,35 g de cristaux de

chlorhydrate de 4-aminobutyramide, soit en rendement de 82 %.

Point de fusion : 136-138°C.

Les spectres IR et RMN sont conformes à la structure du produit recherché.

Exemple 7

15 g de chlorhydrate de 4-aminobutyronitrile sont mis en suspension dans 150 ml d'acide propionique. Le mélange est porté à 50°C puis on fait passer au sein de la suspension un léger courant de gaz chlorhydrique anhydre. Le milieu est maintenu 9 heures dans ces conditions.

Après concentration sous pression réduite au quart de son volume initial le concentrat, traité selon les mêmes conditions que dans l'exemple 6, conduit à 13 g de chlorhydrate de 4-aminobutyramide, soit un rendement de 75 %.

Point de fusion : 136-137°C.

Les spectres IR et RMN sont conformes à la structure du produit recherché.

REVENDICATIONS

1. Procédé de préparation du chlorhydrate de 4-aminobutyramide, ou gabamide, caractérisé en ce qu'il consiste essentiellement à effectuer une hydrolyse ménagée du chlorhydrate de 4-aminobutyronitrile.

2. Procédé selon la revendication 1, caractérisé en ce que l'hydrolyse ménagée du chlorhydrate de 4-aminobutyronitrile est effectuée dans un acide, aqueux ou anhydre, en présence d'acide chlorhydrique ou de gaz chlorhydrique.

3. Procédé selon la revendication 2, caractérisé par le fait que l'hydrolyse est réalisée dans l'acide chlorhydrique aqueux concentré, en l'absence de tout autre acide.

4. Procédé selon la revendication 3, caractérisé en ce que l'hydrolyse est effectuée dans de l'acide chlorhydrique à 22° Bé (d = 1,19).

5. Procédé selon la revendication 2, caractérisé en ce que l'hydrolyse est effectuée dans un acide choisi de préférence parmi l'acide formique, l'acide acétique ou l'acide propionique, seul ou en mélange, anhydre ou non, en présence d'acide chlorhydrique aqueux ou de gaz chlorhydrique anhydre.

6. Procédé selon l'une des revendications 1 à 5, caractérisé par le fait que l'hydrolyse est réalisée à une température allant de 15 à 55°C.

7. Procédé selon la revendication 6, caractérisé par le fait que la durée de réaction est de 60 heures à 15°C à environ 10 heures à 55°C.

8. Procédé selon l'une des revendications 1 à 7, caractérisé en ce que le produit est isolé par addition d'un agent désolubilisant comme l'isopropanol, l'acétone ou un mélange des deux.

9. Procédé selon l'une des revendications 1 à 8, caractérisé en ce que pour isoler le produit on effectue une concentration partielle du milieu, sous pression réduite, puis on ajoute un solvant de désolubilisation.

0110754

Office européen des brevets

# RAPPORT DE RECHERCHE EUROPEENNE

Numéro de la demande

EP 83 40 2133

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| Y | FR-A-1 343 206 (MERCK) * résumé * --- | 1 | C 07 C 103/183<br>C 07 C 102/08 |
| Y | FR-A-1 325 982 (UNION CARBIDE) * résumé * --- | 1-4 | |
| D,Y | EP-A-0 029 890 (DEGUSSA) * revendications * --- | 1 | |
| D,Y | EP-A-0 056 082 (DEGUSSA) * revendications * ----- | 1 | |
| | | | **DOMAINES TECHNIQUES RECHERCHES (Int. Cl. 3)** |
| | | | C 07 C 103/00<br>C 07 C 102/00 |

Le présent rapport de recherche a été établi pour toutes les revendications

| Lieu de la recherche | Date d'achèvement de la recherche | Examinateur |
|---|---|---|
| LA HAYE | 07-02-1984 | MOREAU J.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82